**Europäisches Patentamt**

**European Patent Office**

**Office européen des brevets**

(19)

(11) Veröffentlichungsnummer: **0 208 180**
**B1**

# EUROPÄISCHE PATENTSCHRIFT

(12)

(45) Veröffentlichungstag der Patentschrift:
**16.03.88**

(21) Anmeldenummer: **86108398.8**

(22) Anmeldetag: **19.06.86**

(51) Int. Cl.⁴: **C 07 C 19/045,** C 07 C 17/156,
B 01 J 27/122

(54) **Verfahren zur Herstellung von 1,2-Dichlorethan durch Oxichlorierung von Ethylen an Kupfer enthaltenden Trägerkatalysatoren.**

(30) Priorität: **22.06.85 DE 3522474**

(43) Veröffentlichungstag der Anmeldung:
**14.01.87 Patentblatt 87/3**

(45) Bekanntmachung des Hinweises auf die Patenterteilung:
**16.03.88 Patentblatt 88/11**

(84) Benannte Vertragsstaaten:
**BE DE NL SE**

(56) Entgegenhaltungen:
**DE-A-2 651 974**
**DE-B-2 356 549**

(73) Patentinhaber: **BASF Aktiengesellschaft, Carl-Bosch- Strasse 38, D-6700 Ludwigshafen (DE)**

(72) Erfinder: **Eichhorn, Hans- Dieter, Dr.,
Buchnerstrasse 13, D-6700 Ludwigshafen (DE)**
Erfinder: **Schneehage, Hans Henning, Dr., Plauser Strasse 10, D-6719 Weisenheim (DE)**
Erfinder: **Cordemans, Luc, Dr., Jozef Muislaan 13, B-2080 Kapellen (DE)**
Erfinder: **Hebgen, Werner, Dr., Kurpfalzstrasse 20, D-6907 Nussloch (DE)**
Erfinder: **Jaeckh, Christof, Dr., Ludolf- Krehl-Strasse 39 a, D-6900 Heidelberg (DE)**
Erfinder: **Mross, Wolf Dieter, Dr., Anselm-Feuerbach- Strasse 21, D-6710 Frankenthal (DE)**

**Beschreibung**

Die Erfindung betrifft ein Verfahren zur Herstellung von 1,2-Dichlorethan durch Oxichlorierung von Ethylen an einem Kupfer- und Alkalimetalle enthaltenden Trägerkatalysator. Sie betrifft auch den hierbei verwendeten Katalysator.

Die Oxichlorierung von Ethylen durch Umsetzung mit Chlorwasserstoff und Sauerstoff ist ein bekanntes, großtechnisch ausgeübtes Verfahren. Bei einem solchen Verfahren wird ein Katalysator verwendet, der Kupfer-(II)-chlorid bzw. Kupferoxichlorid auf einem porösen, feuerfesten Träger wie aktiviertes Aluminiumoxid, Siliziumdioxid, Aluminiumsilikat oder Kieselgur enthält. Der Katalysator kann außerdem noch Zusatzstoffe wie Alkalimetallchloride, Metallchloride der Seltenen Erden und andere Metallverbindungen enthalten, die die gewünschte Reaktion aktivieren und/oder unerwünschte Nebenreaktionen verhindern.

Bei der Durchführung dieses Verfahrens wird der erforderliche Sauerstoff, gegebenenfalls in Form von Luft, gemeinsam mit HCl und Ethylen über eine einzige, durch geeignete Wärmeabführung möglichst isotherm betriebene Katalysatorschicht geleitet, vgl. US-A-3 184 515. Die Umsetzung des Ethylens mit Chlorwasserstoff und Luft bzw. Sauerstoff kann nach der GB-A-1 104 666 auch in mehreren hintereinander angeordneten Reaktoren durchgeführt werden. Dabei kann sowohl Luft bzw. Sauerstoff wie auch Chlorwasserstoff in mehrere Ströme aufgeteilt jedem Reaktor getrennt zugeführt werden.

Eine weitere Variante dieser Verfahren besteht darin, das aus der Reaktionszone austretende Reaktionsgemisch teilweise mit frischem Ethylen, Chlorwasserstoff und Luft bzw. Sauerstoff vermischt in die Reaktionszone zurückzuführen. Gegebenenfalls können 1,2-Dichlorethan und Wasser aus dem Reaktionsgemisch vor der Rückführung vollständig oder zum Teil abgetrennt werden, vgl. DE-A-3 130 552.

Bei der Durchführung eines Oxichlorierungsverfahrens ist es besonders wichtig, die Temperatur in der Katalysatorschicht zu kontrollieren. Da die Oxichlorierungsreaktion stark exotherm ist, besteht die Gefahr, daß sich unerwünscht hohe, lokalisierte Temperaturzonen ("hot-spots") in der Katalysatorschicht bilden. Diese können zur Zerstörung des Katalysators führen, wodurch der Katalysator zerfällt und der Druckverlust über der Katalysatorschüttung ansteigt. Dies führt zur Leistungsverminderung der Oxichlorierungsanlage, so daß der Katalysator unter hohen Kosten ausgewechselt bzw. erneuert werden muß. Außerdem führen zu hohe Temperaturzonen in der Katalysatorschicht in erheblichem Maße zu unerwünschten Nebenreaktionen, wodurch die Ausbeuten an 1,2-Dichlorethan herabgesetzt werden.

Es wurden viele Maßnahmen vorgeschlagen, solche "hot-spots" zu verhindern oder mindestens zu verringern. Beispielsweise schlägt die DE-B-2 356 549 vor, durch Verdünnen der Einsatzstoffe mit Inertgas, durch Variation der Verhältnisse der Einsatzstoffe, durch Verdünnen der Katalysatorteilchen mit Inertmaterial oder durch Variation der Teilchengröße der Katalysatoren und/oder der Inertteilchen die Temperatur zu kontrollieren.

Als Inertmaterial zur Verdünnung der Katalysatoren wurden Siliziumdioxid, Aluminiumoxid, Graphit, Glas oder keramische Teilchen in den verschiedensten Formen wie Tabletten, Kugeln, Ringe oder Stränge vorgeschlagen. Das Verhältnis von Katalysator zu Inertmaterial kann im Reaktor variiert und den Erfordernissen der Reaktion angepaßt werden. Zweckmäßig liegt am Reaktoranfang die höchste Konzentration an Inertmaterial vor und nimmt dann zum Auslaß des Reaktors allmählich oder in Stufen ab. Deshalb können im Reaktor auch mehrere Zonen mit unterschiedlichem Verhältnis von Katalysator zu Inertmaterial vorliegen.

Es können auch Katalysatoren mit erniedrigter Aktivität eingesetzt werden. Dadurch können im Reaktor in Bereichen, wo hohe Temperaturspitzen auftreten, Zonen niedriger Aktivität geschaffen werden, die solche hotspots wirksam verringern. Bei einstufiger Fahrweise des Oxichlorierungsverfahrens nimmt dann die Aktivität des Katalysators von Reaktoranfang zum Reaktorende hin zu, bei mehrstufiger Fahrweise kann die Aktivität auch noch von Stufe zu Stufe zunehmen. Zusätzlich können die einzelnen Katalysatoren auch noch mit Inertmaterial abgemischt werden, vgl. GB-A-1 104 666.

Zur Herstellung von Katalysatoren mit unterschiedlicher Aktivität wurden verschiedene Maßnahmen vorgeschlagen. Eine der Möglichkeiten zur Steuerung der Aktivität besteht darin, die Konzentrationen an Kupfer-(II)-chlorid, das üblicherweise in Mengen zwischen 1 und 20 Gew.-% Kupfer, bezogen auf die Katalysatorgesamtmasse, vorliegt, zu variieren. Katalysatoren mit niedriger Aktivität weisen deshalb in der Regel niedrigere Kupferkonzentrationen als solche mit hoher Aktivität auf, so daß nach dem Vorschlag der GB-A-1 104 666 die Konzentration der Kupferverbindung auf dem Trägermaterial in Produktstromrichtung erhöht wird.

Eine weitere Möglichkeit, die Aktivität des Katalysators zu dämpfen, besteht darin, den Katalysator mit Alkalimetallsalzen, bevorzugt in Form ihrer Chloride wie Kaliumchlorid, Natriumchlorid, Lithiumchlorid und weiterer Alkalimetallchloride zu dotieren. Bis zu 3 Gew.-% an Alkalimetall kann im Katalysator enthalten sein, bei höheren Konzentrationen ist nach den Feststellungen der DE-A-3 130 552 die Aktivität dann meist zu gering. Zur Herstellung von Katalysatoren mit niedriger Aktivität können nach dieser Lehre auch beide Möglichkeiten, niedrigere Kupferkonzentrationen und die Dotierung mit Alkali, miteinander kombiniert werden.

Während am Reaktoreingang also am günstigsten Katalysatorzonen mit niedriger Aktivität verwendet werden sollten, muß die Aktivität des Katalysators in Produktstromrichtung ansteigen und am Reaktorende, wo auf Grund der nur noch niedrigen Reaktantenpartialdrücke die Gefahr von Temperaturspitzen nur noch sehr gering ist, am höchsten sein, damit ein möglichst hoher Umsatz der Einsatzstoffe gewährleistet ist.

Als Träger für den Katalysator, dessen Menge den Gesamtkatalysator zu 100 % ergänzt, sind hauptsächlich Aluminiumoxid, Siliziumdioxid, Mischungen aus Aluminiumoxid und Kieselsäure sowie Aluminiumsilikate

geeignet. Bevorzugt wird γ-Aluminiumoxid verwendet, dessen spezifische Oberfläche nach BET zwischen 80 und 350 m2/g bei Porenvolumina im Bereich von 0,4 bis 1,0 cm3/g liegt. Bezüglich der Aktivität spielt auch die gewählte Form des Trägers eine gewisse Rolle. Als Trägerformen kommen Tabletten, Kugeln oder Ringe in Betracht, vgl. DE-B-2 356 549.

Die Herstellung des Katalysators erfolgt durch ein- oder mehrmaliges Imprägnieren des Trägers mit einer die Aktivkomponenten und die Promotoren enthaltenden Lösung. Daran schließt sich ein Trocknen bei Temperaturen zwischen 100 und 500°C in Luft, in Inertgasen wie z. B. Stickstoff oder in Mischungen aus beiden an. Zum Aufbringen der Aktivkomponenten wurden verschiedene Methoden vorgeschlagen:

Die Methode der GB-A-1 104 666 besteht darin, den Träger mit wäßrigen Lösungen aus Kupfer-(II)-chlorid und gegebenenfalls Alkalimetallsalzen und weiteren Promotoren wie z. B. Metallchloride der Seltenen Erden zu tränken und anschließend zu trocknen. Nach der DE-B-2 356 549 wird vorgeschlagen, das Trägermaterial mit einer wäßrigen Lösung zu tränken, die neben Alkaliionen Kupfer-(II)-oxichlorid und Chlorwasserstoff im Gewichtsverhältnis von Kupferionen zu Chlorwasserstoff von 2 : 1 bis 1 : 1 enthält. Daran schließt sich ein Behandeln bei Temperaturen bis 300°C an, wobei wesentliche Mengen an Sauerstoff auszuschließen sind.

Die Durchführung des Oxichlorierungsverfahrens selbst in ein- oder in mehrstufiger Fahrweise mit oder ohne Gasrückführung ist dem Fachmann bekannt. Die Temperaturen in den Reaktoren sollten im allgemeinen zwischen 200 und 320°C liegen, die Drücke zwischen 2 und 10 bar.

Nachteilig an den Katalysatoren der bekannten Oxichlorierungsverfahren ist die Tatsache, daß trotz aller genannten Maßnahmen doch mit der Zeit ein Katalysatorzerfall auftritt, der zu einem Anstieg des Druckverlustes über der Katalysatorschüttung führt, so daß der Katalysator ausgewechselt werden muß. Außerdem werden mit diesen Katalysatoren nur unvollständige Umsätze der Einsatzstoffe erzielt, was zeigt, daß die Aktivität der Katalysatoren, die eine hohe Aktivität im Bereich niedriger Reaktantenpartialdrücke aufweisen sollen, noch ungenügend ist. Darüberhinaus werden stets auch gewisse Mengen an unerwünschten Nebenprodukten, dabei vor allem Ethylchlorid, gebildet, was die Ausbeuten an 1,2-Dichlorethan verringert und das nach der Abtrennung von 1,2-Dichlorethan durch kostspielige Maßnahmen wieder in ungefährliche Stoffe umgewandelt werden muß.

Der Erfindung liegt somit die Aufgabe zugrunde, ein Verfahren zur Herstellung von 1,2-Dichlorethan durch Oxichlorierung von Ethylen sowie einem Oxichlorierungskatalysator zu schaffen, mit denen die Herstellung von 1,2-Dichlorethan über lange Zeit hinweg mit hoher Leistung, hoher Ausbeute und geringem Anfall an Nebenprodukten möglich ist.

Diese Aufgabe wird gelöst mit einem Verfahren der genannten Art, das in Gegenwart eines Katalysators durchgeführt wird, der auf einem üblichen Träger Kupfer-(II)-chlorid in einer Konzentration von 1 bis 20 Gew.-% Kupferionen, bezogen auf das Gesamtgewicht des Katalysators, enthält, wobei das Verfahren dadurch gekennzeichnet ist, daß der Katalysator erhalten wurde durch Tränken des Trägermaterials mit einer wäßrigen Lösung, die Kupfer-(II)-chlorid und Chlorwasserstoff im Gewichtsverhältnis von Kupferionen zu Chlorwasserstoff von 13 : 1 bis 130 : 1 enthält.

Bevorzugte Ausführungsformen des erfindungsgemäßen Verfahrens sind Gegenstand der Unteransprüche 2 bis 5. Der erfindungsgemäße Katalysator weist die Merkmale des Anspruchs 6, bevorzugt auch der Ansprüche 7 und 8, auf.

Die Tränklösung wird hergestellt durch Lösen von Kupfer-(II)-chlorid, welches die chemische Formel $CuCl_2 - 2H_2O$ aufweist, und gegebenenfalls Kaliumchlorid und gewünschtenfalls weiterer Promotoren in Wasser und Zugabe von Chlorwasserstoff, vorzugsweise in Form einer wäßrigen Lösung, so daß in der Tränklösung ein Gewichtsverhältnis von Kupferionen zu Chlorwasserstoff von 13 : 1 bis 130 : 1, vorzugsweise von 25 : 1 bis 50 : 1, vorliegt. Die Reihenfolge, in welcher die einzelnen Komponenten gelöst werden, ist nicht entscheidend. Wichtig ist, daß das erfindungsgemäße Verhältnis von Kupferionen zu Chlorwasserstoff in der Tränklösung vorliegt.

Enthält die Tränklösung zuviel Chlorwasserstoff, etwa nach dem Stand der Technik (DE-B-2 356 549) ein Verhältnis von Kupferionen zu Chlorwasserstoff von 2 : 1 bis 1 : 1, so ergeben sich bei der Durchführung des Oxichlorierungsverfahrens wesentlich niedrigere Standzeiten und auch geringere Umsätze und Ausbeuten als unter Verwendung der erfindungsgemäßen Katalysatoren.

Wird andererseits nach dem Stand der Technik (GB-A-1 104 666) eine Tränklösung ohne jeglichen Zusatz von Chlorwasserstoff verwendet, so werden bei der Durchführung des Oxichlorierungsverfahrens erheblich niedrigere Umsätze und Ausbeuten, verbunden mit niedrigeren Standzeiten, erreicht als unter Verwendung der erfindungsgemäßen Katalysatoren.

Als Träger für dieses Katalysatorsystem können die herkömmlichen und bekannten Träger verwendet werden. Vorzugsweise wird γ-Al2O3 verwendet, das eine BET-Oberfläche zwischen 100 und 250 m2/g aufweist. Als Form für den Träger kommen die üblichen und bekannten geometrischen Formen in Betracht, besonders bevorzugt werden Ringe, Tabletten und Kugeln.

Die Konzentration an Kupfer-(II)-chlorid auf dem Katalysator liegt zwischen 1 und 20 Gew.-% Kupfer, bevorzugt zwischen 2 und 13, insbesondere 3 und 9 Gew.-%. Die Konzentration selbst muß je nach der gewünschten Aktivität des Katalysators und den Erfordernissen der Reaktion eingestellt werden. Als Promotoren können zusätzlich Alkalimetallsalze, bevorzugt die Chloride und Chloride der Seltenen Erden, mit auf den Träger aufgebracht werden. Besonders Kaliumchlorid wird als Promotor bevorzugt und zwar in einem molekularen Verhältnis von Kupfer zu Kalium von 1 : 1 bis 10 : 1. Um die gewünschte Menge an Aktivkomponente und Promotor aufzubringen, kann der Träger ein- oder mehrmahls imprägniert werden. Die Aktivkomponente und die Promotoren müssen nicht unbedingt gleichzeitig auf den Träger aufgebracht werden. Es kann z. B. zunächst Cu-(II)-chlorid und Chlorwasserstoff und dann die Promotoren bzw. umgekehrt aufgebracht werden. Beim Tränken des Trägers mit der Lösung soll die Gesamtmenge an Flüssigkeit etwa der Wasseraufnahmefähigkeit des Trägers entsprechen. Die Technik der Tränkung und der Trocknung des Trägers ist dem Fachmann bekannt und beispielsweise in der DE-B-2 356 549 beschrieben. Die Trocknung des getränkten Katalysators soll bei Temperaturen zwischen 100 und 500°C, vorzugsweise bis 300°C erfolgen und wird zweckmäßigerweise unter Stickstoff durchgeführt. Ein kleiner Anteil von bis etwa 3 % an Sauerstoff kann aber ohne Nachteile in Kauf genommen werden.

Die Durchführung des Oxichlorierungsverfahrens unter Verwendung des erfindungsgemäßen Katalysatorsystems kann ein- oder auch mehrstufig erfolgen. Bei mehrstufiger Durchführung können auch einzelne Einsatzstoffe, z. B. Luft bzw. Sauerstoff oder Chlorwasserstoff, aufgeteilt und einzelnen Stufen getrennt zugeführt werden.

Das aus der Reaktionszone austretende Reaktionsgemisch kann auch bei ein- oder mehrstufiger Fahrweise teilweise mit frischem Ethylen, Chlorwasserstoff und Luft bzw. Sauerstoff vermischt in die Reaktionszone zurückgeführt werden. Gegebenenfalls kann aus dem Reaktionsgemisch vor der Rückführung vollständig oder zum Teil Dichlorethan und Wasser abgetrennt werden. Die Temperaturen in den Katalysatorschüttungen sollten bei den üblichen Werten zwischen 200 und 320°C und die Drücke bei bis etwa 10 bar liegen.

Zur Vermeidung von zu hohen Temperaturspitzen in der Katalysatorschüttung kann es bei der Durchführung des Verfahrens unter Verwendung des erfindungsgemäßen Katalysatorsystems zweckmäßig sein, die Aktivität des Katalysators abzustufen, so daß die Aktivität bei einstufiger Durchführung im Reaktor bzw. bei mehrstufiger Durchführung zumindest in einer Stufe in Produktstromrichtung zunimmt. Die Abstufung der Katalysatoraktivität kann durch bekannte Maßnahmen erfolgen, z. B. durch Änderung der Kupfer-(II)-chlorid-Konzentration des Katalysators, durch Dotierung des Katalysators mit Alkali oder durch Zugabe von Verdünnungsmitteln.

Die folgenden Beispiele sollen die Erfindung näher erläutern:

## Beispiel 1

Herstellung eines erfindungsgemäßen Katalysators:

Imprägniert werden 1000 g $\gamma$-Aluminiumoxid-Ringtabletten mit den Abmessungen: Höhe 5 mm, Außendurchmesser 5 mm, Wandstärke 1,6 mm. Die Wasseraufnahme des Trägers beträgt 0,48 $cm^3$/g. Der Träger wird mit 880 $cm^3$ einer wäßrigen Lösung imprägniert, die 319,8 g $CuCl_2 \cdot 2H_2O$, 24,2 g KCl und 11,2 g konzentrierte Salzsäure (37 %-ig) enthält. Danach wird unter Stickstoff 17 Stunden bei 70°C, dann 3 h bei 120°C und danach 3 h bei 210°C getrocknet.

## Vergleichsbeispiel 1

Herstellung eines Katalysators nach dem Stand der Technik

a) 1000 g $\gamma$-$Al_2O_3$-Ringtabletten werden mit 880 $cm^3$ einer wäßrigen Lösung imprägniert, die 319,8 g $CuCl_2 \cdot 2H_2O$ und 24,2 g KCl enthält. Die Tränkung und die Trocknung entsprechen dem Verfahren des Beispiels 1.

b) 1000 g $\gamma$-$Al_2O_3$-Ringtabletten werden mit 880 $cm^3$ einer wäßrigen Lösung getränkt, die Kupferoxichlorid und Chlorwasserstoff in einem Gewichtsverhältnis von Kupferionen zu Chlorwasserstoff von 1 : 1 enthält, weiterhin sind in der Imprägnierlösung 24,2 g KCl enthalten. Die relativen Mengen von Kupfer und Kalium pro Trägereinheit sowie die Tränkung und die Trocknung entsprechen dem Beispiel 1 und dem Vergleichsbeispiel 1a).

## Beispiel 2

Prüfung der Katalysatoren

a) Aktivitäts- und Selektivitätstests

90 $cm^3$ Katalysator werden in ein Edelstahlrohr mit 64 mm Innendurchmesser eingefüllt und pro Stunde mit einer Gasmischung beaufschlagt, die 0,986 mol Chlorwasserstoff, 0,493 mol Ethylen und 0,246 mol Sauerstoff enthält, wobei Sauerstoff in Form von Luft zugeführt wird. Ein Teil des aus der Katalysatorschüttung austretenden Gases wird mit frischem Einsatzgas vermischt und in die Reaktionszone zurückgeführt. Das Verhältnis von rückgeführtem Reaktionsgas zu frischem Einsatzgas beträgt 20 : 1. Die Temperatur in der Katalysatorschicht beträgt 200°C und der Druck 1 bar. Nach Erreichen eines stationären Betriebszustandes werden dem aus der Reaktionszone austretenden Gas Proben entnommen und daraus der Umsatz und die Ausbeute an 1,2-Dichlorethan bestimmt. Als Maß für den Umsatz wird der prozentuale Anteil des umgesetzten Chlorwasserstoffs angegeben, als Maß für die Ausbeute die Selektivität in Form des prozentualen Anteils vom umgesetzten Ethylen, der zu 1,2-Dichlorethan, zu Ethylchlorid sowie zu sonstigen Produkten umgewandelt wurde.

b) Standzeittest

Zur Prüfung der Standzeit des Katalysators werden 25 g Katalysator in einen Edelstahlreaktor mit 10 mm Innendurchmesser eingefüllt. Der Druckverlust der Katalysatorschüttung wird bei Raumtemperatur bei einer Beaufschlagung mit 600 l Stickstoff pro Stunde und einem Druck vor der Schüttung von 1,5 m Wassersäule gemessen.

Danach wird der Reaktor mit einer Salzschmelze auf 250°C aufgeheizt und der Katalysator pro Stunde mit einem Gas beaufschlagt, welches 16,9 l Chlorwasserstoff, 15,9 l Ethylen und 32,9 l Luft enthält. Nach 6 Tagen wird der Reaktor unter Stickstoff abgekühlt und der Druckverlust unter den obigen Bedingungen erneut gemessen. Anschließend wird der Katalysator ausgebaut und der Anteil an Katalysator bestimmt, der zu Teilchen zerfallen ist, die kleiner als 1 mm sind.

Als Maß für die Standzeit des Katalysators wird der prozentuale Anstieg des Druckverlustes sowie der prozentuale Anteil des Feinanteils im ausgebauten Katalysator angegeben.

Die Ergebnisse der Versuche sind in der nachstehenden Tabelle zusammengefaßt.

**Tabelle**

|  | Erfindungsgemäß | Stand der Technik nach Vergleichsbeispiel 1 | |
| --- | --- | --- | --- |
|  |  | a) | b) |
| HCl-Umsatz in % | 29,2 | 22,4 | 27,8 |
| Mol-% des umgesetzten Ethylens zu |  |  |  |
| 1,2-Dichlorethan | 99,84 | 99,62 | 99,42 |
| Ethylchlorid | 0,14 | 0,26 | 0,50 |
| Rest | 0,02 | 0,12 | 0,08 |
| Anstieg des Druckverlustes in % | 27 | 42 | 135 |
| Feinanteil kleiner als 1 mm Gew.-% | 7,5 | 7,7 | 11,2 |

Die Ergebnisse zeigen, daß bei der Durchführung des Oxichlorierungsverfahrens unter Verwendung der erfindungsgemäßen Katalysatoren bessere Resultate erzielt werden als unter Verwendung von Katalysatoren, die nach dem Stand der Technik hergestellt wurden.

Sowohl die Aktivität wie auch die Ausbeute an 1,2-Dichlorethan sind unter Verwendung der erfindungsgemäßen Katalysatoren höher, während der Anfall an Nebenprodukten geringer ist. Besonders die Standzeit ist bei Verwendung des erfindungsgemäßen Katalysators ebenfalls höher, was sich im geringeren Anstieg des Druckverlustes der Katalysatorschüttung sowie im kleineren Feinanteil zeigt.

**Patentansprüche**

1. Verfahren zur Herstellung von 1,2-Dichlorethan durch Oxichlorierung von Ethylen in Gegenwart eines Katalysators, der auf einem üblichen Träger Kupfer-(II)-chlorid in einer Konzentration von 1 bis 20 Gew.-% Kupferionen, bezogen auf das Gesamtgewicht des Katalysators, enthält, dadurch gekennzeichnet, daß der Katalysator erhalten wurde durch Tränken des Trägermaterials mit einer wäßrigen Lösung, die Kupfer(II)-chlorid und Chlorwasserstoff im Gewichtsverhältnis von Kupferionen zu Chlorwasserstoff von 13 : 1 bis 130 : 1 enthält.

2. Verfahren nach Anspruch 1, dadurch gekennzeichnet, daß die wäßrige Lösung Kupfer-(II)-chlorid und Chlorwasserstoff im Gewichtsverhältnis von Kupferionen zu Chlorwasserstoff von 25 : 1 bis 50 : 1 enthält.

3. Verfahren nach einem der Ansprüche 1 oder 2, dadurch gekennzeichnet, daß die wäßrige Lösung zusätzlich Kaliumchlorid im Gewichtsverhältnis Kupferionen zu Kaliumionen von 1 : 1 bis 10 : 1 enthält.

4. Verfahren nach einem der Ansprüche 1 bis 3, dadurch gekennzeichnet, daß das Kupfer-(II)-chlorid auf dem Träger in einer Konzentration von 2 bis 13 Gew.-% Kupferionen, bezogen auf das Gesamtgewicht des Katalysators, vorliegt.

5. Verfahren nach einem der Ansprüche 1 bis 4, dadurch gekennzeichnet, daß die Oxichlorierung in einem oder mehreren hintereinander angeordneten Reaktoren, gegebenenfalls unter Rückführung von aus der Reaktionszone austretendem und von Dichlorethan und Wasser ganz oder teilweise befreitem Abgas, durchgeführt wird, wobei mindestens einer der Reaktoren unterteilt ist in verschiedene Reaktionszonen und die Konzentration an Kupfer auf dem Trägermaterial in den verschiedenen Reaktionszonen unterschiedlich ist und in Produktstromrichtung zunimmt.

6. Oxichlorierungskatalysator für die Herstellung von 1,2-Dichlorethan, enthaltend Kupfer-(II)-chlorid auf einem üblichen Träger in einer Konzentration von 1 bis 20 Gew.-% Kupferionen, bezogen auf das Gesamtgewicht des Katalysators, erhältlich durch Tränken des Trägermaterials mit einer wäßrigen Lösung, die Kupfer-(II)-chlorid und Chlorwasserstoff im Gewichtsverhältnis von Kupferionen zu Chlorwasserstoff von 13 : 1 bis 130 : 1, insbesondere von 25 : 1 bis 50 : 1, enthält.

7. Oxichlorierungskatalysator nach Anspruch 6, erhältlich durch Tränken des Trägermaterials mit einer wäßrigen Lösung, die zusätzlich Kaliumchlorid im Gewichtsverhältnis Kupferionen zu Kaliumionen von 1 : 1 bis 10 : 1 enthält.

8. Oxichlorierungskatalysator nach einem der Ansprüche 6 oder 7, enthaltend Kupfer-(II)-chlorid auf dem Träger in einer Konzentration von 2 bis 13 Gew.-% Kupferionen, bezogen auf das Gesamtgewicht des Katalysators.

## Claims

1. A process for the preparation of 1,2-dichloroethane by oxychlorination of ethylene in the presence of a catalyst which contains, on a conventional carrier, copper (II) chloride in a concentration corresponding to 1-20 % by weight, based on the total weight of the catalyst, of copper ions, wherein the catalyst was obtained by impregnating the carrier with an aqueous solution which contains copper (II) chloride and hydrogen chloride in such amounts that the weight ratio of copper ions to hydrogen chloride is from 13 : 1 to 130 : 1.

2. A process as claimed in claim 1, wherein the aqueous solution contains copper (II) chloride and hydrogen chloride in such amounts that the weight ratio of copper ions to hydrogen chloride is from 25 : 1 to 50 : 1.

3. A process as claimed in claim 1 or 2, wherein the aqueous solution additionally contains potassium chloride in such an amount that the weight ratio of copper ions to potassium ions is from 1 : 1 to 10 : 1.

4. A process as claimed in any of claims 1 to 3, wherein the copper (II) chloride is present on the carrier in such a concentration that the amount of copper ions is from 2 to 13 % by weight, based on the total weight of the catalyst.

5. A process as claimed in any of claims 1 to 4, wherein the oxychlorination is carried out in one or more reactors arranged in series, if desired with recycling of exit gas which leaves the reaction zone and has been completely or partly freed from dichloroethane and water, and one or more reactors are divided into different reaction zones, and the concentrations of copper on the carrier differ in the various reaction zones and increase in the direction of product flow.

6. An oxychlorination catalyst for the preparation of 1,2-dichloroethane, containing copper (II) chloride on a conventional carrier in such a concentration that the amount of copper ions is from 1 to 20 % by weight, based on the total weight of the catalyst, and obtainable by impregnating the carrier with an aqueous solution which contains copper (II) chloride and hydrogen chloride in such amounts that the weight ratio of copper ions to hydrogen chloride is from 13 : 1 to 130 : 1, in particular from 25 : 1 to 50 : 1.

7. An oxychlorination catalyst as claimed in claim 6, obtainable by impregnating the carrier with an aqueous solution which additionally contains potassium chloride in such an amount that the weight ratio of copper ions to potassium ions is from 1 : 1 to 10 : 1.

8. An oxychlorination catalyst as claimed in claim 6 or 7, containing copper (II) chloride on a carrier in such a concentration that the amount of copper ions is from 2 to 13 % by weight, based on the total weight of the catalyst.

## Revendications

1. Procédé de préparation du dichlor-1,2 éthane par l'oxychloration de l'éthylène en présence d'un catalyseur comprenant, sur un support usuel, du chlorure cuivrique dans une concentration de 1 à 20 % du poids total du catalyseur d'ions cuivre, caractérisé en ce que ce catalyseur a été préparé par l'imprégnation de la matière support avec une solution aqueuse, contenant du chlorure cuivrique et de l'hydrogène chloré dans un rapport pondéral des ions cuivre à l'hydrogène chloré compris entre 13 : 1 et 130 : 1.

2. Procédé suivant la revendication 1, caractérisé en ce que la solution aqueuse contient le chlorure cuivrique et l'hydrogène chloré dans un rapport pondéral des ions cuivre à l'hydrogène chloré compris entre 25 : 1 et 50 : 1.

3. Procédé suivant l'une des revendications 1 et 2, caractérisé en ce que la solution aqueuse contient en plus du chlorure de potassium dans un rapport pondéral des ions cuivre aux ions potassium compris entre 1 : 1 et 10: 1.

4. Procédé suivant l'une des revendications 1 à 3, caractérisé en ce que le chlorure cuivrique est déposé sur le support en une concentration de 2 à 13 % du poids total du catalyseur d'ions cuivre.

5. Procédé suivant l'une des revendications 1 à 4, caractérisé en ce que l'oxychloration est réalisée dans un

6

réacteur ou dans plusieurs réacteurs successifs, éventuellement en y recyclant les produits gazeux résiduels quittant la zone de réaction après l'élimination complète ou partielle du dichlor-éthane et de l'eau, l'un au moins des réacteurs étant subdivisé en plusieurs zones de réaction, dans lesquelles la concentration du cuivre sur la matière support est variable, allant croissante dans la direction d'écoulement du produit.

6. Catalyseur d'oxychloration pour la préparation du dichlor-1,2 éthane, contenant sur un support usuel du chlorure cuivrique en une concentration entre 1 et 20 % du poids total du catalyseur d'ions cuivre, préparé par imprégnation de la matière support avec une solution aqueuse, qui contient du chlorure cuivrique et de l'hydrogène chloré dans un rapport pondéral des ions cuivre à l'hydrogène chloré compris entre 13 : 1 et 130 : 1, en particulier entre 25 : 1 et 50 : 1.

7. Catalyseur d'oxychloration suivant la revendication 6, caractérisé en ce qu'il est préparé par l'imprégnation de la matière support avec une solution aqueuse, qui contient en outre du chlorure de potassium, le rapport pondéral des ions cuivre aux ions potassium étant compris entre 1 : 1 et 10 : 1.

8. Catalyseur d'oxychloration suivant l'une des revendications 6 et 7, caractérisé en ce que le support contient du chlorure cuivrique en une concentration de 2 à 13 % du poids total du catalyseur d'ions cuivre.